# EUROPEAN PATENT SPECIFICATION

(11) **EP 2 110 118 B1**
(45) Date of publication and mention of the grant of the patent: **19.11.2014**
(21) Application number: 08305104.5
(22) Date of filing: 15.04.2008
(51) Int. Cl.: A61L 9/03, A61K 8/35, A61K 8/37, A61L 9/01, A61Q 15/00, C11C 5/00, C11D 17/00, A61L 9/05, A61L 9/12, A61L 9/14, C11D 3/50, C11D 7/26, C11D 3/00, A61K 8/49

(54) **Malodour reducing composition and uses thereof**
Zusammensetzung zur Verringerung von schlechten Gerüchen und Verwendungen dafür
Composition de réduction des mauvaises odeurs et utilisations de celle-ci

(43) Date of publication of application: 21.10.2009
(73) Proprietor: Takasago International Corporation, Tokyo-to 144-8721 (JP)
(72) Inventor: Fraser, Stuart, Little Neston, CH64 4DH (GB); Warr, Jonathan, 75017, PARIS (FR); Bassereau, Maud, 92250, LA GARENNE COLOMBES (FR)
(74) Representative: Nevant, Marc

(56) References cited:
- WO-A-01/38440
- WO-A-01/43784
- WO-A-02/34226
- WO-A-97/26926
- WO-A-2004/043169
- JP-A- 7 330 619
- JP-A- 8 275 997
- JP-A- 2005 350 354

## Description

### Technical Field

This invention is concerned with the use of certain perfume ingredients to counteract malodours, especially human body odours, bathroom odours and odours from domestic waste. The invention also relates to perfumes and consumer products which incorporate such perfume ingredients.

### Background

The problem of malodours has been recognised for many years, and numerous methods have been developed to overcome these where they occur. Perfumes are commonly used as malodour counteractants either alone or in combination with other materials such as absorbents, oxidants and solubilisers.

Malodours are usually caused by particularly odorous substances such as sulphur compounds e.g. hydrogen sulphide, and low molecular weight thiols and thioethers, nitrogen containing compounds such as ammonia and amines including heterocyclic compounds such as pyrazines and indoles and oxygen containing compounds such as short chain fatty acids and steroids. These malodorous compounds are often created by the action of micro-organisms e.g. on food and drink during digestion, on sweat produced in the axilla or on the feet, on waste food awaiting disposal or animal waste such as cat litter. As natural products the malodours are themselves complex mixtures, not only of several compounds within each of the above chemical categories but also including members of all the categories to different degrees depending on the source of the odour and micro-organisms involved. Consequently preventing, countering, reducing or destroying malodour is not straightforward.

Various methods have been developed to counter or reduce malodour beyond odour masking with strong fragrance. Activated carbon and zeolites have been used as absorbents for malodours but these are not suited for incorporation into many products.

Antimicrobial agents such as zinc compounds and Triclosan (2', 4, 4'-trichloro-2-hydroxydiphenyl ether) are used to kill microorganisms involved in producing malodours, but many are non-specific and can lead to imbalances in the natural microflora.

US 4,304,679, US 4,322,308, US 4,278,658 and US 4,134,838 all describe fragrances which exhibit a deodorant action when applied to human skin or when included in a laundry product. These are believed to work by any or all of a variety of mechanisms: reducing the vapour pressure of malodorous compounds, inhibiting the enzymes which generate the malodours and combining with the malodour to modify the perceived odour note.

US 4,719,105 describes the use of cyclomethanols and esters as low odour materials which can be used to mask malodours without having unduly strong fragrances themselves.

US2004/0147416 describes the use of cyclodextrins to trap malodorous molecules. However malodours tend to become stronger over time as the population of micro-organisms grows and malodour production increases.

A different approach is to inhibit specific biological pathways. Compounds which act more specifically include saturated C₃-C₁₀ dioic acids which inhibit a sweat decomposing esterase as described in DE 4,343,265.

US 6,183,731 describes the use of agents which are inhibitors of 5α-reductase, such as octadecendioic acid as a preventative agent for body malodour.

US 5,395,555 teaches an aqueous cleaning composition for carpets, rugs, and textiles, particularly useful in reducing malodour of urine stains containing one of a group of selected metal sequestrants which is thought to deny heavy metals essential to the micro-organisms' development.

However as mentioned earlier malodours are complex and such approaches may not counter all the components of the malodour, so it is more likely that they modify the nature of the odour which again may be a worthwhile benefit but is only a partial solution to the problem.

Aldehydes are sometimes cited as malodour counteractants. US 4,906,454 describes piroctone acid and a perfume with a high concentration of aldehydes and/or ketones for use in deodorants in preventing body malodours. US 6,177,070 describes a deodorant product containing at least two different aldehydes present in the form of addition compounds such as acetals and hemi-acetals and an unsaturated compound.

US 5,676,163 describes two classes of aldehydes which act additively or synergistically to reduce the malodour of tobacco smoke. US 2005/187123 and US 2005/187124 describe compositions to be incorporated into solid and liquid carpet cleaner and deodourising products to remove malodours from carpets. US 2002/0010447 describes two categories of aldehydes for malodour reduction in fibrous absorbents for bodily fluids. The categories of aldehydes which are claimed to work synergistically are aliphatic aldehydes and aldehydes which have an sp2 hybridised carbon in the alpha position to the aldehyde carbon.

### Summary of the Invention

Thus while it is apparent from the prior art that there are many methods which attempt to prevent malodour none are completely satisfactory. The present invention describes a simple and inexpensive way to prevent the development of some of the components of malodour.

The invention concerns a malodour reducing composition for use in consumer products comprising:
A) at least one phenylglycidate of formula (1): 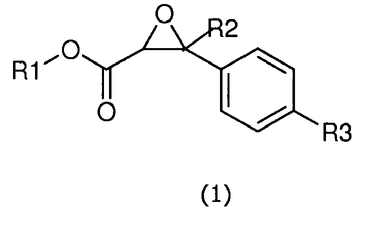 wherein:
   - R1 is a C₁-C₄ branched or linear alkyl group,
   - R2 is hydrogen or methyl, and
   - R3 is hydrogen, a C₁-C₄ branched or linear alkyl group or a methoxy group, and
B) at least one 1,2 diketone of formula (2) or (3): 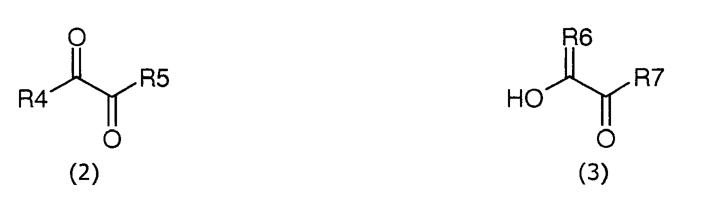 wherein:
   - R4, R5 and R7 may be independently, a C₁-C₅ linear or branched alkyl or alkenyl group;
   - R6 is a (C₁-C₅) alkylidene;
   - R4 and R5 may also form a C₄-C₇ saturated or unsaturated alicyclic or heterocyclic ring structure, which may be mono-substituted or poly-substituted by (C₁-C₄) alkyl groups;
   - R6 and R7 may also form a C₄-C₇ unsaturated, alicyclic or heterocyclic ring structure which may be mono-substituted or poly-substituted by (C₁-C₄) alkyl groups;
      the weight ratio of glycidate A to 1,2 diketone B being from 1:99 to 99:1.

The invention concerns the use as a malodour reducing agent of the composition comprising:
A) at least one phenylglycidate of formula (1): 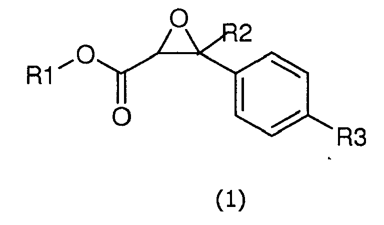 wherein:
   - R1 is a C₁-C₄ branched or linear alkyl group,
   - R2 is hydrogen or methyl, and
   - R3 is hydrogen, a C₁-C₄ branched or linear alkyl group or a methoxy group, and
B) at least one 1,2 diketone of formula (2) or (3): 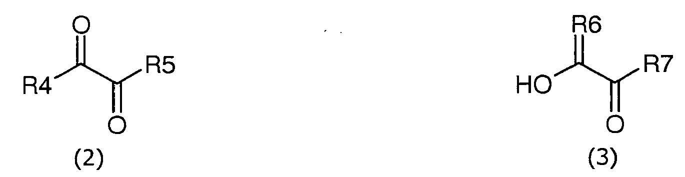 wherein:
   - R4, R5 and R7 may be independently, a C₁-C₅ linear or branched alkyl or alkenyl group;
   - R6 is a (C₁-C₅) alkylidene;
   - R4 and R5 may also form a C₄-C₇ saturated or unsaturated alicyclic or heterocyclic ring structure, which may be mono-substituted or poly-substituted by (C₁-C₄) alkyl groups;
   - R6 and R7 may also form a C₄-C₇ unsaturated, alicyclic or heterocyclic ring structure which may be mono-substituted or poly-substituted by (C₁-C₄) alkyl groups;
      the weight ratio of glycidate A to 1,2 diketone B being from 1:99 to 99:1.

Another object of the invention concerns a consumer product containing a malodour reducing composition in an amount of 0.01% to 1.5% by weight of the total composition of said consumer product, said malodour reducing composition being a combination of A) and B) as defined above, wherein said consumer product is selected from personal care products, laundry detergents and conditioners, household cleaners and air care products.

### Detailed Description of the Invention

In this specification, all percentages quoted are weight percent unless otherwise stated. At the first mention compound chemical names are followed by the Chemical Abstracts reference number.

It has been found that phenylglycidates used in perfumery can counter many malodours: such as human body odours, kitchen cooking, toilet, urine, faeces, tobacco smoke and animal litter. However the phenylglycidates have relatively strong and specific odours so that they are not suited to be used at high level in a wide range of fragrances. Another group of chemical compounds which also counters malodours are 1,2-diketones. The diketones have a different odour quality and by varying the proportions of phenylglycidates and 1,2-diketones the combination may be used at higher levels in a much wider range of fragrance types. Furthermore these combinations may act synergistically together and with other known malodour countering agents.

### Malodour Reducing Compositions

As used herein the term "malodour reducing compositions" means combinations of phenylglycidates and 1,2 diketones within the invention which reduce the concentration of malodorous compounds in the air and/or reduces the perception of malodour to the human nose as measured by either chemical analysis or sensory methods.

### Malodour Reducing fragrances

As used herein "malodour reducing fragrance" describes a fragrance composition which contains phenylglycidates and 1,2 diketones within the invention and which reduces the concentration of malodorous compounds in the air and/or reduces the perception of malodour to the human nose as measured by either chemical analysis or sensory methods. A malodour reducing fragrance according to the present invention may also contain other fragrance compounds which are known as compounds countering malodours, such as aldehydes and α,β-unsaturated aldehydes. Given the diversity of malodours it can be advantageous to design fragrances to counter specific odours.

### Malodour Reducing ingredients

The main ingredients of the malodour reducing compositions and fragrances of the invention are defined in more detail herein below:

### - Phenylglycidates

Phenylglycidates which have been found to counter malodours, have the general formula (1): 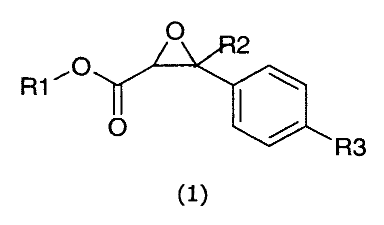 wherein:
- R1 is a C₁-C₄ branched or linear alkyl group,
- R2 is hydrogen or methyl, and
- R3 is hydrogen, a C₁-C₄ branched or linear alkyl group or a methoxy group.

Examples of suitable phenylglycidates include:
- n-butyl 3-methyl-3-phenylglycidate (93963-69-0);
- ethyl 3-(4-methoxyphenyl)glycidate (16546-01-3);
- ethyl 3-(4-methylphenyl)glycidate (52788-71-3);
- ethyl 3-phenylglycidate (121-39-1);
- ethyl 3-methyl-3-phenylglycidate (77-83-8)
- methyl 3-methyl-3-phenylglycidate (99334-18-6).
   Two glycidates which are particularly preferred for inclusion in fragrance compositions are: ethyl-3-phenylglycidate (121-39-1) and ethyl-3-methyl-3-phenylglycidate (77-83-8).

### - 1,2 diketones

Thus, 1,2 diketones which have been found to counteract malodours have the general formula (2) or formula (3), which is the enol form of formula (2): 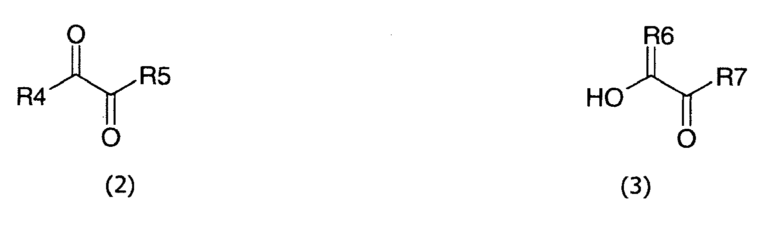 wherein:
- R4, R5 and R7, which may be identical or different, represent a C₁-C₅ linear or branched alkyl or alkenyl group;
- R6 is a (C₁-C₅)alkylidene;
- R4 and R5 may also form a C₄-C₇ saturated or unsaturated alicyclic or heterocyclic ring structure, which may be mono-substituted or poly-substituted by (C₁-C₄) alkyl groups;
- R6 and R7 may also form a C₄-C₇ unsaturated, alicyclic or heterocyclic ring structure which may be mono-substituted or poly-substituted by (C₁-C₄) alkyl groups.

Examples of such 1,2 diketones include:
- 2-hydroxy-4-isopropyt-2,4,6-cycloheptatrien-1-one also known as β-thujaplicine (499-44-5);
- 3-methylcyclopent-2-en-2-ol-1-one (765-70-8) called cyclotene which can also be found listed as the diketone tautomer 3-methyl cyclopentan-1,2-dione with the CAS number (80-71-7);
- 2,3-butanedione (431-03-8);
- 2,3-pentadione (600-14-6);
- 2-ethyl-3-hydroxypyran-4-one (4940-11-8);
- 2-methyl-3-hydroxypyran-4-one (118-71-8);
- 2,3-hexadione (3848-24-6);
- 3,4-hexadione (4437-51-8);
- 2,3-heptadione (96-04-8);
- 3-Ethyl-2-cyclopenten-2-ol-1-one (21835-01-8);
- 3,4-dimethyl-1,2-cyclopentadione (13494-06-9);
- 3,5-dimethyl-1,2-cyclopentadione (13494-07-0);
- Methyl-2-hydroxy-4-methylcyclopent-2-en-1-one (42348-12-9);
- 2-hydroxycyclohex-2-en-1-one (10316-66-2);
- 1- methyl-2,3-cycloheadione (3008-43-3) and
- 1,2 cyclohexadione (765-87-7).

The phenylglycidates and 1,2-diketones used according to the present invention are known commercially available products, or products which may be obtained by processes well known by the person skilled in the art. Bauer et al in Common Fragrance and Flavour Materials, 4th Edition (ISBN 3-527-30364-2) gives brief descriptions and references for the preparation of the more common materials, which are incorporated herein by reference.

Examples of suitable processes for preparing 1,2-diketones are given hereinafter:
X-F Zhao and C Zhang, in Synthesis 2007 p551-557, describes a method for the oxidation of 1,2 diols to 1,2 diketones;
Z Wan, C D Jones, D Mitchell, J Y Pu, and T Y Zhang in J. Org. Chem. 2006, vol 61, p826-828 describes a method for oxidation of alkynes to 1,2 diketones;
J S Yadav, S K Biswas and R Srinivas in Synthesis 2006 p4237-4241 describe a method for the oxidative cleavage of a 1,3 diol to give a 1,2 diketone;
Further references can be found in US patent 4,107,210 assigned to Dow chemicals and references therein which describe commercial preparative routes to 1,2 diketones.
Glycidates are typically prepared either by epoxidation (Prileschaiev's reaction) or condensation (Darzens Glycidic Ester Condensation) reactions. These two synthetic processes for preparing phenylglycidates are described in standard organic chemistry reference books such as Organic Chemistry by I. L. Finar ISBN 0582442214.

The amount of diketones/glycidates required for effective malodour reduction depends on the strength of the malodour, the type of product from which the fragrance is to be delivered, the product dose and the fragrance dose in the product. Given the wide range of products capable of delivering the malodour reducing composition and the range of perfume dosage in consumer products the dosage range of the malodour reducing composition can be from 0.1% to 1.5% of the composition of the consumer product composition.

By composition of the consumer product composition is meant that part of the product which is expended in use; so excluding packaging and containers such as the devices for dispensing air care products, trigger sprays or canisters for pressurized aerosols and holders attached to toilet rims.

For household products for surface application the amount of the malodour reducing composition is preferably between 0.1% and 0.2% of the household product composition.

For products used to treat ambient air the proportion of the malodour reducing composition in the product composition is preferably between 0.01% and 0.05%.

For personal care products for treating skin and hair the proportion of the malodour reducing composition is preferably between 0.1% and 0.2% of the overall personal care product composition.

For laundry detergent and laundrable fabric care products the proportion of the malodour reducing composition is preferably between 0.1% and 1.0% of the product, more preferably between 0.1% and 0.5%.

For personal cleansing products, household cleaning products, including cistern blocks, rim blocks or liquid rim blocks and laundry and fabric care products, the proportion of the fragrance composition of the invention is preferably between 0.01% and 0.05%.

An air care product can comprise a fragrance composition in a proportion of generally between 0.05% and 100%.

Phenylglycidates have relatively strong and specifically fruity odours hence their use in creating certain fragrance types may be limited. The diketones have a different odour quality and by varying the proportions of phenylglycidates and 1,2-diketones the combination may be used at higher levels in a much wider range of fragrance types.

In the malodour reducing composition of the invention the ratio of phenyl glycidate to 1,2-diketone is from 1:9 to 9: 1.

One embodiment of the invention is that the malodour reducing composition should comprise part of a malodour reducing fragrance. Even if the diketones/glycidates are added to the product separately from other fragrance ingredients they are considered part of the fragrance composition. Such a malodour reducing fragrance should contain at least 0.05% in total of the combination of phenylglycidates and 1,2-diketones of the invention, preferably at least 0.1% of the combination of phenylglycidates and 1,2-diketones and more preferably at least 0.2% of the combination of phenylglycidates and 1,2-diketones. While the diketones and glycidates of the invention are especially effective at countering malodours, there are also other malodorants which may be present and for optimal counteraction it may be desirable to add ingredients which assist in malodour counteraction. Depending upon the source of the malodour and on the desired overall odour character of the perfume, there will be an optimal balance between the phenylglycidate and 1,2-diketone combination and adding other perfume ingredients which may offer more general malodour counteraction properties. One category of fragrance ingredients which can be used very effectively in combination with the phenylglycidates and 1,2-diketones of the invention is aldehydes and α,β-unsaturated aldehydes.

### Perfume Composition

In the context of this specification the word "fragrance" is understood to be synonymous with the word "perfume" and the terms "fragrance composition" or "perfume composition" and to refer to a mixture of olfactively active materials providing a pleasant smell. The term "fragrance ingredient" which is also synonymous with the terms "fragrance component", "perfume ingredient" and "perfume component" is taken to mean any individual material which may be an ingredient within a fragrance composition even though that perfume ingredient may itself comprise many individual chemical compounds and possess a pleasant smell.

The fragrance composition to which the malodour reducing composition of the present invention may be added is a mixture, i.e. more than one chemical species, including ingredients which are known to be malodour counteractants. A wide variety of odiferous materials are known for perfumery use, including materials such as alkenes, alcohols, aldehydes, ketones, esters, ethers, nitriles, amines, oximes, acetals, ketals, thiols, thioketones, imines, etc. Without wishing to be limited, the ingredients of the perfume composition will have molecular weights of less than 325 atomic mass units, preferably less than 300 atomic mass units and more preferably less than 275 atomic mass units to ensure sufficient volatility to be noticeable. Furthermore the perfume compounds will have molecular weights greater than 50 atomic mass units, preferably greater than 60 atomic mass units as lower masses may be too volatile. Ingredients of the fragrance compositions will not contain strongly ionizing functional groups such as sulphonates, sulphates, or quaternary ammonium ions, nor halogen atoms.

Fragrance compositions containing the malodour reducing composition of the present invention may optionally contain natural extracts, such as essential oils. Natural extracts are produced by subjecting suitable natural materials such as plant components: leaves, flowers, seeds, roots or stems to an extraction process. The extraction processes are well known to those skilled in the art and many are described in The Essential Oils by E Guenther published in 1949 by D van Nostrand. Essential oils can undergo additional processes to rectify and purify the oils for example by removing the terpene components via a "head cut" and/or removing the wax components via a "tail cut". Such natural extracts include but are not limited to those obtained from citrus species such as: lemons, oranges, mandarin, grapefruit, ugli fruit; from spices such as anise, cinnamon clove, or herbs such as basil, mint, lavender, lavandin, thyme, rosemary, or many varieties of plants such as geranium, various roses, citronella, cypress, eucalyptus, Peru balsam, camphor, sandalwood, ylang and cedarwood and mixtures thereof. A preferred group of natural extracts for the present invention are Amyris oil, cedarwood oil, cocoa absolute, copaiba balsam, menthe oil pays, myrrh resin, patchouli oil, vanillin (absolute) and vetiver oil.

Perfume compositions can be relatively simple in their composition with a minimum of three perfume or fragrance ingredients, including the phenylglycidates and 1,2-diketones, or they can comprise highly complex mixtures of natural and synthetic chemical components, chosen to provide any desired odour. It is preferred if the perfume composition contains more than 5 perfume ingredients, more preferable that they contain more than 10 perfume ingredients. Perfume ingredients are described more fully in S. Arctander, Perfume Flavors and Chemicals. Vol. I and II, Montclair, N.J and in Allured's Flavor and Fragrance Materials-2007 ISBN 978-1-93263326-9 by Allured Publishing Corporation.

### Solvents

Olfactively weak or neutral solvents may comprise part of the product. In the perfume industry it is quite common to dissolve solid fragrance materials in a suitable solvent or to dilute powerful materials, used at low levels, with a solvent to facilitate manufacture. Typical solvents include hydrophobic materials such as benzyl benzoate, isopropyl myristate, dialkyl adipates, citrate esters such as or acetyl tributyl citrate or diethyl phthalate or hydrophilic materials such as isopropanol, propylene glycol or dipropylene glycol or triethyl citrate. The common fragrance solvents which would preferably be used in consumer products containing malodour reducing compositions of this invention are described in S. Arctander, Perfume Flavors and Chemicals. Vols. I and II, Montclair, N.J and in Allured's Flavor and Fragrance Materials-2007 ISBN 978-1-93263326-9 by Allured Publishing Corporation. Moreover solvents can also be beneficial in products, such as citrate esters being used to counter malodours in some personal care deodorant compositions, while other solvents can regulate fragrance evaporation and vapour pressure to achieve a desirable fragrance intensity and rate of fragrance delivery for air care products for example Isopar L^{®} (Exxon Chemicals) and Dowanol DPM glycol ether^{®} (The Dow Chemical Company). In this specification solvents are considered as part of the fragrance composition.

### Consumer Products

As used herein "consumer products" are defined as a functionally effective combination of ingredients which are consumed i.e. used up, in performing the task for which the product was intended such as cleansing, cleaning, deodorising, sanitising, softening, freshening, etc.
Examples of consumer products in which the malodour reducing composition or fragrance composition of the invention may be used are given hereinafter:

### -1) Personal care products

Shampoos, and hair conditioners, as exemplified by Head and Shoulders^{®} or Pantene^{®} (Procter & Gamble) or Elseve^{®} (L'Oreal), shower gels, body wash as exemplified by Radox^{®} (Sara Lee), liquid soap exemplified by Softsoap^{®} (Colgate), soap bars as exemplified by Lux^{®} or Dove^{®} (Unilever), deodorant spray, roll-on and stick deodorants as exemplified by Rexona^{®} or Sure^{®} (Unilever), moist wipes including baby wipes, flushable moist toilet tissues, skin cleansing or refreshing wipes.

### - 2) Laundry detergents and conditioners

Detergent powders, tablets, non soap detergent bars and hard soap bars, concentrated detergent powders, liquid detergents, concentrated liquid detergents, non-aqueous liquid detergents and unit dosing liquid detergent sachets. These may be exemplified by the following brands and variants Tide^{®} and Aerial^{®} (Procter & Gamble) Skip^{®} and Omo^{®} (Unilever) Persil^{®} (Henkel). Fabric softeners or conditioners may be dilute or concentrated aqueous dispersions, or transparent liquids, powders or bars as exemplified by variants of Downy^{®} (Procter & Gamble) Comfort^{®} and Snuggle^{®} (Unilever). Other post wash fabric treatments include tumble drier sheets such a Bounce^{®} (Procter & Gamble) or ironing waters such as Comfort^{®} (Unilever).

### - 3) Household cleaners

Household cleaners include liquid and spray hard surface cleaners which may be used directly on surfaces, liquids which can be diluted e.g. for floor cleaning or cleaning crockery and cooking utensils. Such products are exemplified by Flash^{®} (Procter & gamble), Cif^{®} (Unilever) Fairy^{®} Dishwash liquid (Procter & Gamble) or Pril^{®} (Henkel). Unit dosed solid or liquid sachet or tabletted products such as for machine dishwashing as exemplified by Finish^{®} (Reckitt Benckiser) or Fairy^{®} Active Bursts (Procter & Gamble) and specialist cleaners for glass and mirrors e.g. under the brand Mr Muscle^{®} (SC Johnson) . These products are available in a variety of product forms such as sprays, mousses, liquids, liquids containing suspended solids, impregnated wipes sachets multicompartment sachets and unit dose devices. Bathroom sanitaryware cleaners include limescale cleaners, cistern blocks, rim blocks, liquid rim blocks and hybrid products exemplified by brands such as Harpic^{®} (Reckitt Benckiser). Soft furnishing and fabric cleaners and refreshers include spray products such as Febreze^{®} (Procter & Gamble) and carpet products such as Glade^{®} shake and Vac^{®} (SC Johnson).

### - 4) Air Care products

Air care products include liquid electric powered air freshener devices, aerosol sprays, pump action sprays, fragranced candles, membrane permeation devices, Liquid wick devices, oil based gel fragrances, aqueous gels all of which may be exemplified by the following brands and variants Ambi Pur^{®} (Sara Lee), Glade^{®} and Oust^{®} (SC Johnson) and Air Wick® (Reckitt Benkiser).

Such a diverse group of products contain a wide range of the proportion of fragrance within the formulation and are used at a wide range of product dosages which determine the levels at which the malodour reducing composition of the invention can be employed. Table 1 below gives typical perfume dosages for a range of products in which the invention malodour reducing composition could be used.

**Table 1: Typical Perfume Dosages in Consumer Products**

| Product | Typical Dosage Range (by weight) |
|---|---|
| Electric powered air freshener | 40-100% |
| Ambient temperature membrane devices | 40-100% |
| Ambient temperature fragranced oil gels eg incorporating Sylvaclear PE400 polymer® (Arizona Chemicals) | 40-85% |
| Ambient temperature water based liquid wick | 5-20% |
| Aqueous freshener gels (e.g. carrageenan gels) | 2-10% |
| Liquid rim block, solid rim block, cistern block | 2-12% |
| Fragranced candles | 2-10% |
| Laundry detergent and conditioning products | 0.2-5% |
| Tumble drier sheets | 2-10% |
| Hard surface cleaning products | 0.1-0.5% |
| Toilet soaps, shampoos, hair conditioners, shower gels, moist wipes | 0.05-1.2% |
| Deodorant sprays, roll-ons or sticks | 0.05-1.0% |
| Air freshener trigger sprays and aerosols | 0.2-0.6% |

### Air Fresheners

One form of consumer products are air freshener devices to introduce fragrance to the ambient air in enclosed spaces such as rooms, vehicles, wardrobes, chests, drawers etc. These air freshener products may typically be in the form of sprays, candles, gels, membrane permeation devices, plug-in electrical devices, battery operated devices or liquid wick devices. In the present invention sprays may be aqueous or non-aqueous, pressurised with compressed gas or pump action. Candles and gels may be opaque, translucent or transparent and may be moulded or packaged or contain additional ingredients to enhance their appearance. The plug-in and battery operated devices may include devices that vaporise the fragrance by heat, evaporation, or nebulisation.

Thus the process of dispersing the malodour reducing composition or fragrance containing the malodour reducing composition into ambient air may be by spraying, diffusion, evaporation or nebulisation.

Products for freshening or fragrancing general ambient air countering malodours or repelling insects include devices which have no power source and work by diffusion e.g. through a polymeric membrane, or by electrical heating a wick in contact with a liquid reservoir so that the rate of fragrance can be controlled. Such devices can be electrically powered, so called plug-in fresheners as described in US 6,917,754 or US 7,223,361 or WO2007/046692 or battery powered so portable or for use in a motor vehicle. Optionally the device may use an atomizing spray to disperse the fragrance as in WO2007/132140 or contain an electrically powered fan. Malodour reducing fragrance compositions of the present invention can be used advantageously in these freshener devices and compositions.

The present invention also provides a process to impart a malodour reducing fragrance to a substrate which may be skin, hair, soft furnishings, launderable textiles and garments, and hard surfaces including floors, walls, work surfaces toilets and bathroom sanitaryware.

The products from which the malodour reducing fragrances of the invention containing diketones and/or phenylglycidates may be delivered to skin and hair may be in the form of liquids, soft solids, gels, pastes, creams, powders, sprays or impregnated substrates such as pads or wipes. Liquids may include shampoos, conditioners, shower gels, liquid soaps, or lotions. The liquids may be clear opaque or pearlescent, of low viscosity or thickened to a gel-like viscosity. Soft solids include soap bars which includes mixtures of soap with synthetic detergents, such as alkyl isethionates or alkyl ethersulphates and deodorant sticks. Sprays include deodorant or antiperspirant sprays for the skin or hair styling products and may be trigger action or pressurised canister type sprays.

Impregnated substrates include wet wipes for personal hygiene, personal cleansing especially for babies and small children or refreshing. However the malodour reducing compositions of the invention are not intended for use in consumer products which are intended to be ingested such as foodstuffs or drinks, nor those which may be partially ingested as a result of the method of use such as in toothpaste or mouthwashes, nor products which may be accidentally ingested such as chewing gum.

The formulations and ingredients of personal care products in which malodour reducing compositions of the invention may be used are well known to those skilled in the art, reference may be made to the following works :
"Formulating Detergents and Personal Care Products A guide to Product Development" by L Ho Tan Tai, ISBN 1-893997-10-3 published by the AOCS Press, and
"Surface Active Agents and Detergents" Volumes 1 and 2 by Schwartz, Perry and Birch,
   and
"Harry's Cosmeticology" published by CHS Press 8th Edn 2000 ISBN 0-8206-0372-4, and Mc Cutcheon's Detergents and Emulsifiers Published by Allured and
Cosmetic Science and Technology Series Volume 20, Edited by K.Laden, (Marcel Dekker) ISBN 0-8247-1746-5 "Antipersperants and Deodorants" and
Cosmetic Science and Technology Series Volume 17, Ed by Dale H Johnson, ISBN 0-8247-9365-X "Hair and Hair Care" published by Marcel Dekker, as well as to the following patents or patent applications.

Shampoos and Hair Conditioners:
US 6,162,423; US 5,968,286; US 5,935,561; US 5,932,203; US 5,837,661; US 5,776,443; US 5,756,436; US 5,661,118; US 5,618,523; EP 0 018 717; EP 1 009 365; EP 0 200 305.

Wipes: WO 2003/051327; WO 00/04230, EP 1 361 855; US 2005/0008680;

Skin cleansing and care liquids:
US 5,833,999; EP 1 066 827; EP 1 510 201; EP 0 573 229; US 2005/0085405;

### Laundry Products

Malodour reducing compositions of the invention might advantageously be employed in products for domestic cleaning of washable textiles such as clothes, towels, and bed linen. Suitable laundry products include: solid bar, powder, tablet, and liquid detergents, fabric softeners and non-softening fabric conditioning products. Also included are post wash laundry treatment products such as ironing waters, garment refresher sprays and articles impregnated with cleaning or conditioning liquids such as tumble drier sheets. The formulation of laundry products is familiar to those skilled in the art and reference may be made to "Formulating Detergents and Personal Care Products A guide to Product Development" by L Ho Tan Tai, and to "Surface Active Agents and Detergents" Volumes 1 and 2 by Schwartz, Perry and Birch both referenced earlier and also to Volume 67 of the Surfactant Science Science Series Liquid Detergents ISBN 0-8247-9391-9 (Marcel Dekker Inc), as well as to the following patents or patent applications:

Liquid Laundry detergents:
US 5,929,022, US 5,916,862, US 5,731,278, US 5,470,507, US 5,466,802 US 5,460,752, and US 5,458,810.

Detergent Powders and tablets:
WO99/65458, WO99/41353 and EP 1,123,381.

Fabric softeners and conditioners:
US 6,627,598, US 6,335,315, US 5,674,832, US 5,759,990, US 5,877,145, US 5,574,179.

The products from which the malodour reducing fragrances containing diketones and/or phenylglycidates of the invention may be delivered to domestic soft furnishings such as curtains, carpets, or upholstery or hard surfaces such as wooden or tiled floors, walls, windows, kitchen worktops and bathroom sanitaryware, may be in the form of liquids, soft solids, gels, pastes, foams, creams, powders, sprays or impregnated substrates such as pads or wipes. Products may be applied as directly to surfaces, or diluted with water prior to use, or they may be incorporated in a device such as a cistern block or liquid rim block which is activated by flushing the lavatory.

### Lavatory Solid Rim Blocks and Cistern Blocks

One embodiment of the present invention relates to malodour reduction on hard surfaces and sanitaryware. Lavatory blocks including those which are intended, in use, to be located under the rim of a lavatory bowl or urinal such that, during a flushing cycle, water from the cistern flows over the block thereby dissolving a portion of the block are a particular example of a product in which the malodour reducing compounds of the invention can be used. Such blocks are generally known in the art as 'rim blocks' and will be referred to as such or simply as 'blocks' herein. The invention also relates to so-called cistern blocks which are placed in the cistern and dissolve slowly in the water contained therein. It will be appreciated that the solubility characteristics of these two products are quite different, since one is constantly under water while the other has intermittent short term contact with water. However they both contain a surfactant, filler, and fragrance which may contain ingredients of the present invention and optionally bleaching agents, germicides and anti-limescale agents. Typical formulations are taught in WO97/47721, EP0462643, GB 2178442 and US 4874536 .

### Liquid Rim Blocks

Another embodiment of the malodour reducing compounds of this invention relates to lavatory cleaners known as liquid rim blocks. Liquid rim blocks are devices that dispense liquid compositions directly into a lavatory bowl from under the rim of said bowl. Such rim blocks are usually attached by various means, such as hooks and the like, to the rim of the lavatory bowl. Every time a toilet equipped with a rim block is flushed, an amount of composition is dispensed into the lavatory bowl. Examples of liquid rim blocks are given in WO02/40792 which teaches a liquid rim-block device having a suspension means and at least two compartments for active substances. WO 02/04591 teaches a liquid rim block, a lavatory cleaning system comprising a dispenser for dispensing a liquid composition from under the rim of a lavatory bowl, wherein the composition has a viscosity of greater than 2500 mPa.s. Other similar systems are described in EP 0 775 741 and WO01/94520 .

Rim blocks may also comprise combinations of solid and liquid within a single device. Such devices are commercially available for example under the trade name Harpic® from Reckitt Beckiser.

### Liquid Hard Surface Cleaner Formulation

Another specific application of malodour reducing fragrances of the invention relates to hard surface cleaner formulations also known as all purpose cleaners or general purpose cleaners. They are a broad category of products including isotropic liquids, thickened liquids with or without abrasive or as a mousse. They can be used directly from the bottle or after dilution in water. Various delivery methods have been devised for the convenience of the user, some are sprayed onto surfaces from trigger spray bottles, and alternatively they can be poured directly onto surfaces and removed for instance when a lavatory is flushed. They may contain additional ingredients such as acids for limescale removal, biocides for hygiene, or bleaches. Consequently there are a broad range of formulations within this category. Table 2 below summarises the main formulation ingredients and levels (this is taken from Surfactant Science Series Vol 67 Liquid Detergents chapter on Speciality Liquid Household Surface Cleaners p 479 table 4.) In all cases, except when oxidizing bleaches (e.g. sodium hypochlorite or hydrogen peroxide) are incorporated into the formulation, malodour reducing fragrances containing phenylglycidates and 1,2 diketones of this invention may be used as the fragrance within the product.

**Table 2: Typical Formulation Ranges for Ingredients of Household Cleaners**

| Ingredient | Example | Amount wt % |
|---|---|---|
| Anionic surfactant | Alkylbenzene sulphonate, as supplied by Shell as Dobs 055, alkane sulphonate eg Hostaspur SAS60 | 0-35 |
| Nonionic surfactant | Ethoxylated alcohol, e.g.: Neodol 9-11 6EO; mixed ethoxy/propoxy alcohol such as the pluronic series from BASF, amine oxides, alkanolamides and betaines. | 1-35 |
| Hydrotropes | Sodium cumene sulphonate or xylene sulphonate. | 0-10 |
| Builder/sequestrant | Citrates, EDTA salts; phosphonate salts; lactic acid and polyacrylates. | 0-10 |
| Solvent | Lower alcohols; glycol ethers; benzyl alcohol or hydrocarbons, e.g. limonene. | 0.5-50 |
| Disinfectant | Hypochlorite bleach; pine oil; lower alcohols; quaternary ammonium salts. | 0-15 |
| Fragrance; coloring agent; thickening polymer; sequestrant; preservatives | | 0.1-3 |
| Water | | To 100 |

### Carpet cleaners

Carpet cleaners come in several forms: powders, liquids, foams and spray spot treatments. Many of these products have to be used in conjunction with a specific cleaning machine especially if large areas are to be treated. However a desirable attribute of cleaning a carpet is to reduce malodours and leave a pleasant smell, such products generally contain a fragrance. Malodour reducing fragrances of the present invention are both suitable and desirable for application in carpet cleaning products.

### Air Freshener, Textile and Soft Furnishing Deodorizing Sprays

Products for reducing or masking malodours in the air or on textiles and soft furnishings are known, such as Glade^{®} (SC Johnson) and Febreze^{®} (Procter and Gamble). These products are designed to be sprayed into the air or onto surfaces. They can be dispensed either from aerosols with a volatile propellant gas or using a trigger spray which does not require propellant gas. US 2005/0124512 teaches a cyclodextrin containing product in which non fabric discolouring aldehydes are incorporated to react with amines in the air. Nowhere does the patent teach that glycidates or 1,2-diketones either separately or combined might inhibit malodour formation. US2003/0044309 teaches an emulsion composition containing a selection of fragrance aldehydes which counter malodour. Fragrances incorporating glycidate and 1,2-diketones as malodour reducing compounds can be used advantageously in these products.

The following examples are provided to further illustrate the compositions and processes in accordance with the invention.

### Example 1

The malodour reducing capability of individual ingredients, namely diketones and glycidates of the invention is tested according to the following procedure. Procedure
- Add into a 20cm³ headspace vial:
   - 100 µl of a 1% (wt/wt) solution of 1-hexylamine in dipropylene glycol;
   - 7.0 g of demineralised water;
   - 20µl of 1,2-diketone or phenylglycidate;
   - 0.2 g of Emulgin L^{®} (Cognis)
- Mix for 10 minutes at room temperature;
- Equilibrate for 50 minutes at 35°C;
- Measure the headspace concentration of the hexylamine by solid phase microextraction (SPME) on a polydimethylsilicone divinylbenzene fibre (ex Supelco);
- Sample the vapour for 5 minutes at 35°C then desorb onto a 30m HP-5MS GC column at 265°C for 1 minute.
   The heaspace concentration is calculated by comparison with a standard having no malodour reducing fragrance and is reported as the reduction in area percent of the GCMS signal for hexylamine. The results are given in Table 3.

**Table 3: Malodour reduction by compounds of the invention**

| Ingredient (CAS Number) | Malodour reduction % |
|---|---|
| β-Thujaplicine (499-44-5) | 97 |
| 2,3 butanedione (431-03-8) | 82 |
| 3-methylcyclopent-2-en-2-ol-1-one (765-70-8) | 66 |
| 2-methyl-3-hydroxypyran-4-one (118-71-8) | 71 |
| Ethyl 3-methyl-3-phenylglycidate (77-83-8) | 41 |
| Ethyl 3-phenyl glycidate (121-39-1) | 63 |

### Example 2

This example shows the effect of increasing the concentration of a typical diketone β -thujaplicine within fragrance A of table 4 on the malodour compared with the fragrance itself. The procedure is similar to that described in example 1 above except that a 50 µl aliquot of fragrance is used instead of the 20 µl aliquot of the 1,2 diketone or phenylglycidate in example 1. Tthe test fragrance consists partly of fragrance A and partly of a 1,2-diketone, phenylglycidate or a mixture thereof, with part of the diethyl phthalate within fragrance A being replaced by the malodour reducing compounds of the invention. Malodour reduction is measured as the concentration of hexylamine in the vapour phase compared with a control sample which contains fragrance A without any malodour reducing ingredients of the invention.

**Table 4: Formulation of Fragrance A**

| Ingredient | CAS No | % |
|---|---|---|
| Allyl cyclohexyl propionate | 2705-87-5 | 0.2 |
| Allyl heptoate | 142-19-8 | 0.3 |
| Armoise oil | | 0.4 |
| Benzyl acetate | 140-11-4 | 4.1 |
| Benzyl Salicylate | 118-58-1 | 12.0 |
| Dihydromyrcenol | 18479-58-8 | 10.0 |
| Dimethyl anthranilate | 85-91-6 | 0.1 |
| Eugenol | 97-53-0 | 0.3 |
| Florosa | 63500-71-0 | 3.0 |
| Geraniol | 106-24-1 | 4.2 |
| Guaicawood oil | | 0.6 |
| Hedione | 24851-98-7 | 5.0 |
| Cis 3-hexenyl acetate | 35926-04-6 | 0.5 |
| Isobornyl acetate | 125-12-2 | 5.0 |
| Linalool | 78-70-6 | 10.0 |
| Mandarin oil | | 0.6 |
| Methyl ionone gamma A | 127-51-5 | 0.7 |
| Ethylene brassylate | 105-95-3 | 6.0 |
| Patchouli oil de-ironised | | 0.2 |
| Phenyl acetaldehyde Dimethyl acetal | 101-48-4 | 0.2 |
| Phenyl ethyl alcohol | 60-12-8 | 5.2 |
| Rose oxide | 16409-43-1 | 0.2 |
| Styrallyl acetate | 93-92-5 | 0.9 |
| Undecalactone gamma | 104-67-6 | 0.3 |
| Verdox | 88-41-5 | 5.0 |
| Diethyl phthalate | 84-66-2 | 25.0 |

Fragrance A is a citrus floral rose fragrance suitable for use in laundry or personal care products.

**Table 5: Malodour reduction due to dosages of β-thujaplicine**

| Beta thujaplicine in Fragrance A* | Malodour Reduction (%) |
|---|---|
| 1% | 47 |
| 2.5% | 76 |
| 5% | 93 |

| | |
|---|---|
| *β-thujaplicine replaces the equivalent weight of diethyl phthalate in fragrance A. So 1% β-thujaplicine means only 24.0% of diethyl phthalate is used to make up that test sample | |

### Example 3

Example 3 repeats example 2 but with ethyl 3-phenylglycidate instead of β thujaplicine. The results in table 6 show a definite trend in response to increasing the glycidate concentration.

**Table 6 Malodour Reduction due to Dosages of Ethyl 3-phenylglycidate**

| Ethyl 3-phenyl glycidate in Fragrance A** | Malodour Reduction (%) |
|---|---|
| 1% | 31 |
| 5% | 39 |
| 10% | 54 |

| | |
|---|---|
| ** Ethyl 3-phenyl glycidate replaces the equivalent weight of Diethyl Phthalate in fragrance A as described in example 2. | |

### Example 4

Example 4 repeats example 2 but with a range of mixtures of ethyl 3-phenylglycidate and 2-methyl-3-hydroxypyran-4-one (118-71-8). The ratio of ethyl 3-phenylglycidate to 2-methyl-3-hydroxypyran-4-one was used between 9:1 to 1:9 by weight percent, with the total weight of the combination always representing 3.0% of fragrance A. The results show that the combination gives greater malodour reduction than ethyl 3-phenylglycidate at 5% (cf table 6 with table 7) and is equal to the ethyl 3-phenylglycidate at 10% by weight of fragrance A. Furthermore the change in malodour reduction is not particularly sensitive to the ratio of ingredients.

**Table 7: Malodour Reduction due to Combinations of Ethyl Phenylglycidate and 2-Methyl-3-hydroxypyran-4-one**

| Ratio of Ethyl 3-phenylglycidate: 2-methyl-3-hydroxypyran-4-one*** | Malodour Reduction (%) |
|---|---|
| 1:9 | 68 |
| 25:75 | 69 |
| 50:50 | 49 |
| 75:25 | 54 |
| 9:1 | 54 |

| | |
|---|---|
| *** Each mixture of Ethyl 3-phenylglycidate and 2-methyl-3-hydroxypyran-4-one replaces 3.0% of the diethyl phthalate in fragrance A. | |

### Example 5

A perfume composition in the form of a gel for a membrane air care device or an electrically powered air care device is prepared by mixing 40% of the perfume composition of table 8 with 55.95% of Dipropylene glycol monomethyl ether, 4% of fumed hydrophilic silica and 0.05% of polyoxyethylene sorbitan monolaurate.

**Table 8: A Perfume Composition for Gel or Electrically Powered Air Care Devices**

| Ingredient | Chemical name | CAS N ° | % by Weight |
|---|---|---|---|
| HEDIONE | Methyl dihydrojasmonate | 24851-98-7 | 35 |
| | Ethyl-3-methyl-3-phenyl glycidate | 77-83-8 | 1.125 |
| B-THUJAPLICINE (1% in DPG) | | 499-44-5 | 1.875 |
| ORBITONE | Octahydro-2,3,8,8-tetramethyl-2-acetonaphtone | 54464-57-2 | 32.5 |
| DI-HYDRO MYRCENOL | 2,6-dimethyl-7-octen-2-ol | 18479-58-8 | 10.25 |
| MUSK-T | 1,4-Dioxacycloheptadecane-5,17-dione | 105-95-3 | 15 |
| LEVOSANDOL | 2-ethyl-4-(2,2,3-trimethyl-3-cyclopenten-1-yl)-2- buten-1-ol | 28219-61-6 | 2 |
| CITRAL | 3,7-dimethyl-2,6-octadienal (mixture of cis and trans) | 5392-40-5 | 1.25 |
| ALLYL CYCLOHEXYL PROP | Allyl cyclohexanepropionate | 2705-87-5 | 0.25 |
| AMBROXAN | Dodecahydro-3a,6,6,9a-tetramethylnaphtho-(2,1-b)-furane | 3738-00-9 | 0.25 |
| AMBRETONE | 5-cyclohexadecen-1-one | 37609-25-9 | 0.25 |
| HEXENYL ACETATE, CIS-3 | Cis hex-3-en-1-yl acetate | 3681-71-81 | 0.125 |
| | ethyl 2-methylbutyrate | 7452-79-1 | 0.125 |

The malodour reducing composition comprises 3.0% of the fragrance and 1.2% of the final product composition with a ratio of phenylglycidate to 1,2 diketone of 1:1.7. Such a fragrance can be used in devices for the diffusion of ambiance-generating perfumes, which are available from commercial sources, such as those known under the trade names "Glade® Plug-In", "Glade® Wisp", "Reckitt-Benckiser Air-Wick® Mobil", "Air-Wick®", "Ambi-Pur Car®" and "Sara Lee Inspira®".

### Example 6: Perfumed Candle

A perfuming candle was prepared by melting the wax and the other ingredients in table 9 below a water bath at 80° C. and then adding the perfuming oil according to table 10 and mixing until a uniform mixture was obtained, which was then flowed into a mould with a wick.

**Table 9: Candle Composition**

| NAME | % |
|---|---|
| PARAFFIN WAX 52.54 (AIGLON SA) | 22 |
| STEARIC ACID PRISTERENE 9559 (UNIQUIMA) | 20 |
| BEESWAX, CEREWAX A.75 (BAERLOCHER FRANCE) | 8 |
| WHITE VASELINE (AIGLON SA) | 16 |
| LIQUID PARAFFIN (AIGLON SA) | 24 |
| PERFUME POMPOSITION OF TABLE 10 | 10 |

The candle thus formed was then left to cool down for 24 hours. This candle was then burnt so as to diffuse the perfume composition in a stable way during at least 24-36 hours.

**Table 10: Perfuming Oil for Candle**

| Ingredient | Chemical name | CAS N ° | % |
|---|---|---|---|
| HEDIONE | Methyl dihydrojasmonate | 24851-98-7 | 29.0 |
| LINALOOL | 3,7-dimethyl-1,6-octadien-3-ol | 78-70-6 | 18.2 |
| MUSK-T | 1,4-Dioxacycloheptadecane-5,17-dione | 105-95-3 | 12.3 |
| VERTENEX | 4-(1,1-dimethylethyl)cyclohexanol acetate | 32210-23-4 | 12.3 |
| DI-HYDRO MYRCENOL | 2,6-dimethyl-7-octen-2-ol | 18479-58-8 | 7.4 |
| VERDOX | o-t-Butylcyclohexyl acetate | 88-41-5 | 6.1 |
| | Ethyl-3-methyl-3-phenyl glycidate | 121-39-1 | 2.0 |
| Veltol plus | 2-ethyl-3-hydroxypyran-4-one | 4940-11-8 | 0.3 |
| CITRAL | 3,7-dimethyl-2,6-octadienal (mixture of cis and and trans) | 5392-40-5 | 3.1 |
| ALLYL CYCLOHEXYL PROP | Allyl cyclohexyl-3 propionate | 2705-87-5 | 2.5 |
| CITRONELLYL NITRILE, L | (3R)-3,7-dimethyloct-6-enenitrile | 51566-62-2 | 2.4 |
| LEVOSANDOL | 2-ethyl-4-(2,2,3-trimethyl-3-cyclopenten-1-yl)-2- buten-1-ol | 28219-61-6 | 1.2 |
| ORBITONE | Octahydro-2,3,8,8-tetramethyl-2-acetonaphthone | 54464-57-2 | 2.5 |
| AMBROXAN | Dodecahydro-3a,6,6,9a-tetramethylnaphtho (2,1-b) furane | 3738-00-9 | 0.2 |
| DIMETHYL BENZYL CARBINYL BUTYRATE | | 10094-34-5 | 0.2 |
| ETHYL 2-METHYLBUTYRATE | | 7452-79-1 | 0.1 |
| CLONAL | 1-cyanoundecane | 2437-25-4 | 0.1 |
| ETH VANILLIN | 3-ethoxy-4-hydroxybenzaldehyde | 121-32-4 | 0.1 |

| | | | |
|---|---|---|---|
| The fragrance contains 2.3% of a combination of phenylglycidate and 1,2 diketone in a ratio of phenylglycidate:1,2 diketone of 6.67:1 and the malodour reducing composition of the invention comprises 0.23% of the final product composition. | | | |

### Example 7: A Gel Air Freshener

Table 11 gives the formulation of a water based gel air freshener to which the fragrance of table 8 can be added for a malodour countering effect.

**Table 11**

| Ingredient | % |
|---|---|
| Deionised water | To 100 |
| Carageenan gum | 3.0 |
| Guar Gum | 0.3 |
| Preservative | 0.3 |
| Fragrance of table 8 | 4.0 |

Add the carageenan gum and Guar gum to water with continuous mixing. Heat the water to 80°C with steady mixing until the polymers have dissolved. Allow to cool and add the fragrance and preservative at 60°C with mixing. After 10 minutes pour into container.

The phenylglycidate and 1,2 diketone malodour reducing composition comprise 3% of the fragrance of table 8 and thereby comprise 0.12% of the final air freshener gel.

### Example 8: Water Containing Aerosol Air Freshener

Table 12 gives the formulation of an aqueous air freshener spray into which fragrances of the invention such as the fragrance of table 8 can advantageously employed to reduce malodour.

**Table 12: An Aqueous Air Freshener Composition**

| Ingredient | % |
|---|---|
| Fragrance of table 8 corrected to 100% | 0.40 |
| Propellant (Propane/Butane) | 30.00 |
| Monosodium dihydrogen phosphate/ Sodium Hydroxide pH buffer | q.s. Approx 1% |
| Sorbitan mono-oleate | 1.00 |
| Water | To 100% |

### Example 9: Liquid Rim block

Table 13 gives an example of a toilet liquid rim block in which the fragrance of table 8 can be advantageously used to reduce malodour.

**Table 13: Liquid Rim Block Composition**

| Ingredient | Wt % |
|---|---|
| Emulgin HF70 (supplied by Cognis) | 16.7 |
| 1,2 Propylene glycol | 4.0 |
| Fragrance of table 8 | 6.0 |
| Kathon CG (supplied by Seppic) | 0.005 |
| Natrosol 250MR (supplied by Hercules) | 11.7 |
| Dyes and other minors | q.s. |
| Water | To 100 |

## Claims

1. A malodour reducing composition consisting of:
A) at least one phenylglycidate of formula (1): 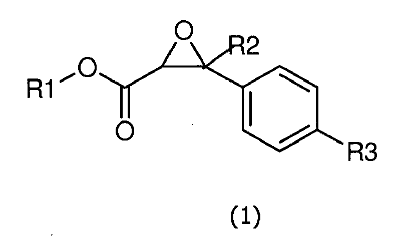 wherein:
- R1 is a C₁-C₄ branched or linear alkyl group,
- R2 is hydrogen or methyl, and
- R3 is hydrogen, a C₁-C₄ branched or linear alkyl group or a methoxy group, and
B) at least one 1,2 diketone of formula (2) or (3): 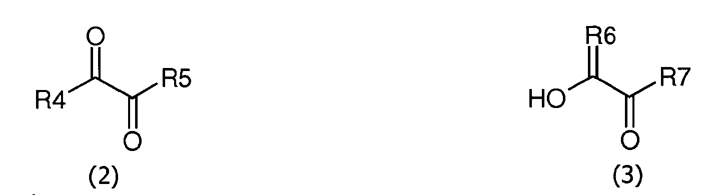 wherein:
- R4, R5 and R7 may be independently, a C₁-C₅ linear or branched alkyl or alkenyl group;
- R6 is a (C₁-C₅) alkylidene;
- R4 and R5 may also form a C₄-C₇ saturated or unsaturated alicyclic or heterocyclic ring structure, which may be mono-substituted or poly-substituted by (C₁-C₄)alkyl groups;
- R6 and R7 may also form a C₄-C₇ unsaturated, alicyclic or heterocyclic ring structure which may be mono-substituted or poly-substituted by (C₁-C₄) alkyl groups;
the weight ratio of glycidate A to 1,2 diketone B being from 1:9 to 9:1.

2. The malodour reducing composition according to claim 1, wherein the phenylglycidate is selected from ethyl-3-phenylglycidate (121-39-1), ethyl-3-methyl-3-phenylglycidate (77-83-8) or mixtures thereof.

3. The malodour reducing composition according to claim 1 or claim 2, wherein the 1,2 diketone is selected from β thujaplicine (499-44-5), 3-methylcyclopent-2-en-2-ol-1-one (765-70-8), 2-ethyl-3-hydroxypyran-4-one (4940-11-8), 2-methyl-3-hydroxypyran-4-one (118-71-8), ethyl cycloentenolone (21835-01-8), 3,4-dimethyl-1,2-cyclopentadione (13494-06-9), 3,5-dimethyl-1,2-cyclopentadione (13494-07-0), methyl-2-hydroxy-4-methylcyclopent-2-en-1-one (42348-12-9), 2-hydroxycyclohex-2-en-1-one (10316-66-2), 1-methyl-2,3-cyclohexadione (3008-43-3), 1,2 cyclohexadione (765-87-7) and mixtures thereof.

4. The malodour reducing composition according to claim 3, wherein the 1,2 diketone is selected from β thujaplicine (499-44-5), 2-ethyl-3-hydroxypyran-4-one (4940-11-8), 2-methyl-3-hydroxypyran-4-one (118-71-8) and mixtures thereof.

5. A consumer product which comprises a malodour reducing composition in an amount of 0.01% to 1.5% by weight of the total composition of said consumer product, wherein the maladour reducing composition is a combination of:
A) at least one phenylglycidate of formula (1): 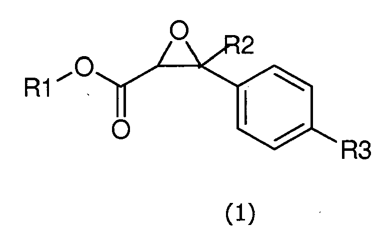 wherein:
- R1 is a C₁-C₄ branched or linear alkyl group,
- R2 is hydrogen or methyl, and
- R3 is hydrogen, a C₁-C₄ branched or linear alkyl group or a methoxy group, and
B) at least one 1,2 diketone of formula (2) or (3): 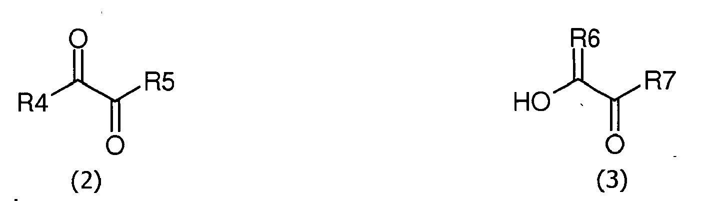 wherein:
- R4, R5 and R7 may be independently, a C₁-C₅ linear or branched alkyl or alkenyl group;
- R6 is a (C₁-C₅) alkylidene;
- R4 and R5 may also form a C₄-C₇ saturated or unsaturated alicyclic or heterocyclic ring structure, which may be mono-substituted or poly-substituted by (C₁-C₄) alkyl groups;
- R6 and R7 may also form a C₄-C₇ unsaturated, alicyclic or heterocyclic ring structure which may be mono-substituted or poly-substituted by (C₁-C₄) alkyl groups;
the weight ratio of glycidate A to 1,2 diketone B being from 1:9 to 9:1;
and wherein said consumer product is selected from personal care products, laundry detergents and conditioners, household cleaners and air care products.

6. The consumer product according to claim 5, wherein the phenylglycidate is selected from ethyl-3-phenylglycidate (121-39-1), ethyl-3-methyl-3-phenylglycidate (77-83-8) or mixtures thereof.

7. The consumer product according to claim 5 or claim 6, wherein the 1,2 diketone is selected from β thujaplicine (499-44-5), 3-methylcyclopent-2-en-2-ol-1-one (765-70-8), 2-ethyl-3-hydroxypyran-4-one (4940-11-8), 2-methyl-3-hydroxypyran-4-one (118-71-8), ethyl cycloentenolone (21835-01-8), 3,4-dimethyl-1,2-cyclopentadione (13494-06-9), 3,5-dimethyl-1,2-cyclopentadione (13494-07-0), methyl-2-hydroxy-4-methylcyclopent-2-en-1-one (42348-12-9), 2-hydroxycyclohex-2-en-1-one (10316-66-2), 1-methyl-2,3-cyclohexadione (3008-43-3), 1,2 cyclohexadione (765-87-7) and mixtures thereof.

8. The consumer product according to claim 7, wherein the 1,2 diketone is selected from β thujaplicine (499-44-5), 2-ethyl-3-hydroxypyran-4-one (4940-11-8), 2-methyl-3-hydroxypyran-4-one (118-71-8) and mixtures thereof.

9. The use of a combination of:
A) at least one phenylglycidate of formula (1): 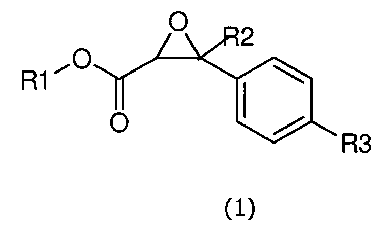 wherein:
- R1 is a C₁-C₄ branched or linear alkyl group,
- R2 is hydrogen or methyl, and
- R3 is hydrogen, a C₁-C₄ branched or linear alkyl group or a methoxy group, and
B) at least one 1,2 diketone of formula (2) or (3): 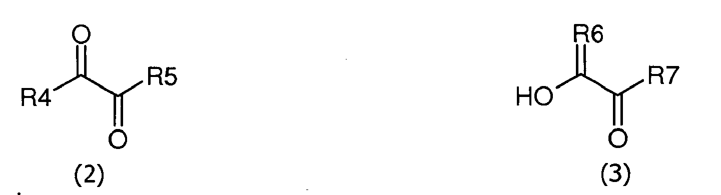 wherein:
- R4, R5 and R7 may be independently, a C₁-C₅ linear or branched alkyl or alkenyl group;
- R6 is a (C₁-C₅) alkylidene;
- R4 and R5 may also form a C₄-C₇ saturated or unsaturated alicyclic or heterocyclic ring structure, which may be mono-substituted or poly-substituted by (C₁-C₄) alkyl groups;
- R6 and R7 may also form a C₄-C₇ unsaturated, alicyclic or heterocyclic ring structure which may be mono-substituted or poly-substituted by (C₁-C₄) alkyl groups;
the weight ratio of glycidate A to 1,2 diketone B being from 1:9 to 9:1, as a malodour reducing agent.

10. The use according to claim 9, wherein the phenylglycidate is selected from ethyl-3-phenylglycidate (121-39-1), ethyl-3-methyl-3-phenylglycidate (77-83-8) or mixtures thereof.

11. The use according to claim 9 or claim 10, wherein the 1,2 diketone is selected from β thujaplicine (499-44-5), 3-methylcyclopent-2-en-2-ol-1-one (765-70-8), 2-ethyl-3-hydroxypyran-4-one (4940-11-8), 2-methyl-3-hydroxypyran-4-one (118-71-8), ethyl cycloentenolone (21835-01-8), 3,4-dimethyl-1,2-cyclopentadione (13494-06-9), 3,5-dimethyl-1,2-cyclopentadione (13494-07-0), methyl-2-hydroxy-4-methylcyclopent-2-en-1-one (42348-12-9), 2-hydroxycyclohex-2-en-1-one (10316-66-2), 1-methyl-2,3-cyclohexadione (3008-43-3), 1,2 cyclohexadione (765-87-7) and mixtures thereof.

12. The use according to claim 11, wherein the 1,2 diketone is selected from thujaplicine (499-44-5), 2-ethyl-3-hydroxypyran-4-one (4940-11-8), 2-methyl-3-hydroxypyran-4-one (118-71-8) and mixtures thereof.

## Patentansprüche

1. Geruchsreduzierende Zusammensetzung, bestehend aus
A) wenigstens einem Phenylglycidat der Formel (1): 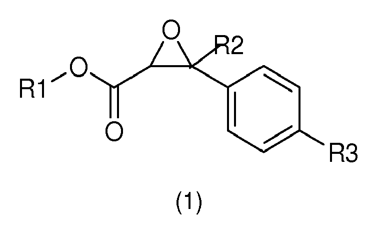 wobei
- R1 eine C₁-C₄-verzweigte oder lineare Alkylgruppe ist,
- R2 ein Wasserstoff oder ein Methyl ist und
- R3 ein Wasserstoff, eine C₁-C₄-verzweigte oder lineare Alkylgruppe oder eine Methoxygruppe ist, und
B) wenigstens ein 1,2-Diketon der Formel (2) oder (3): 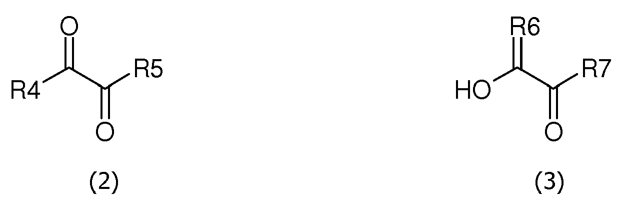 wobei
- R4, R5 und R7 unabhängig voneinander eine C₁-C₅-lineare oder verzweigte Alkyl- oder Alkenylgruppe sein können,
- R6 ein (C₁-C₅)-Alkyliden darstellt,
- R4 und R5 auch eine C₄-C₇-gesättigte oder ungesättigte alicyclische oder heterocyclische Ringstruktur bilden können, die durch (C₁-C₄)₋Alkylgruppen monosubstituiert oder polysubstituiert sein kann,
- R6 und R7 auch eine C₄-C₇-ungesättigte, alicyclische oder heterocyclische Ringstruktur bilden können, die durch (C₁-C₄)-Alkylgruppen monosubstituiert oder polysubstituiert sein kann,
wobei das Gewichtsverhältnis von Glycidat A zu 1,2-Diketon B zwischen 1:9 und 9:1 liegt.

2. Geruchsreduzierende Zusammensetzung nach Anspruch 1, wobei das Phenylglycidat aus Ethyl-3-phenylglycidat (121-39-1), Ethyl-3-methyl-3-phenylglycidat (77-83-8) oder Mischungen davon ausgewählt ist.

3. Geruchsreduzierende Zusammensetzung nach einem der Ansprüche 1 oder 2, wobei das 1,2-Diketon aus β-Thujaplicin (499-44-5), 3-Methylcyclopent-2-en-2-ol-1-on (765-70-8), 2-Ethyl-3-hydroxypyran-4-on (4940-11-8), 2-Methyl-3-hydroxypyran-4-on (118-71-8), Ethylcycloentenolon (21835-01-8), 3,4-Dimethyl-1,2-cyclopentadion (13494-06-9), 3,5-Dimethyl-1,2-cyclopentadion (13494-07-0), Methyl-2-hydroxy-4-methylcyclopent-2-en-1-on (42348-12-9), 2-Hydroxycyclohex-2-en-1-on (10316-66-2), 1-Methyl-2,3-cyclohexadion (3008-43-3), 1,2-Cyclohexadion (765-87-7) und Mischungen davon ausgewählt ist.

4. Geruchsreduzierende Zusammensetzung nach Anspruch 3, wobei das 1,2-Diketon aus β-Thujaplicin (499-44-5), 2-Ethyl-3-hydroxypyran-4-on (4940-11-8), 2-Methyl-3-hydroxypyran-4-on (118-71-8) und Mischungen davon ausgewählt ist.

5. Ein Verbrauchsprodukt, das eine geruchsreduzierende Zusammensetzung in einer Menge von 0,01 bis 1,5 Gewichts-% der Gesamtzusammensetzung des Verbrauchsproduktes umfasst, wobei die geruchsreduzierende Zusammensetzung eine Kombination ist von:
A) wenigstens einem Phenylglycidat der Formel (1): 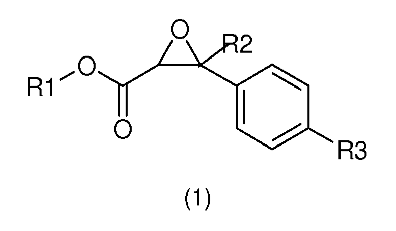 wobei
- R1 eine C₁-C₄-verzweigte oder lineare Alkylgruppe ist,
- R2 ein Wasserstoff oder ein Methyl ist und
- R3 ein Wasserstoff, eine C₁-C₄-verzweigte oder lineare Alkylgruppe oder eine Methoxygruppe ist, und
B) wenigstens ein 1,2-Diketon der Formel (2) oder (3): 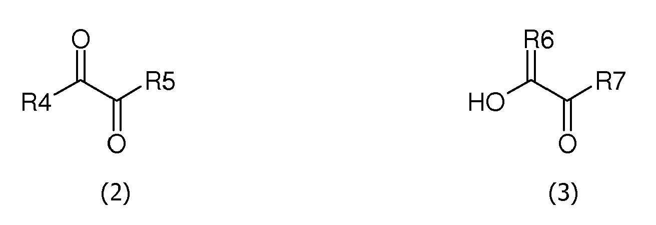 wobei
- R4, R5 und R7 unabhängig voneinander eine C₁-C₅-iineare oder verzweigte Alkyl- oder Alkenylgruppe sein können,
- R6 ein (C₁-C₅)-Alkyliden darstellt,
- R4 und R5 auch eine C₄-C₇-gesättigte oder ungesättigte alicyclische oder heterocyclische Ringstruktur bilden können, die durch (C₁-C₄)_Alkylgruppen monosubstituiert oder polysubstituiert sein kann,
- R6 und R7 auch eine C₄-C₇-ungesättigte, alicyclische oder heterocyclische Ringstruktur bilden können, die durch (C₁-C₄)-Alkylgruppen monosubstituiert oder polysubstituiert sein kann,
wobei das Gewichtsverhältnis von Glycidat A zu 1,2-Diketon B zwischen 1:9 und 9:1 liegt, und
wobei das Verbrauchsprodukt aus Körperpflegeprodukten, Waschmitteln, Haarspülungen, Haushaltsreinigern und Luftreinigungsprodukten ausgewählt ist.

6. Verbrauchsprodukt nach Anspruch 5, wobei das Phenylglycidat aus Ethyl-3-phenylglycidat (121-39-1), Ethyl-3-methyl-3-phenylglycidat (77-83-8) oder Mischungen davon ausgewählt ist.

7. Verbrauchsprodukt nach Anspruch 5 oder 6, wobei das 1,2-Diketone aus β-Thujaplicin (499-44-5), 3-Methylcyclopent-2-en-2-ol-1-on (765-70-8), 2-Ethyl-3-hydroxypyran-4-on (4940-11-8), 2-Methyl-3-hydroxypyran-4-on (118-71-8), Ethylcycloentenolon (21835-01-8), 3,4-Dimethyl-1,2-cyclopentadion (13494-06-9), 3,5-Dimethyl-1,2-cyclopentadion (13494-07-0), Methyl-2-hydroxy-4-methylcyclopent-2-en-1-on (42348-12-9), 2-Hydroxycyclohex-2-en-1-on (10316-66-2), 1-Methyl-2,3-cyclohexadion (3008-43-3), 1,2-cyclohexadion (765-87-7) oder Mischungen davon ausgewählt ist.

8. Verbrauchsprodukt nach Anspruch 7, wobei das 1,2-Diketon aus β-Thujaplicin (499-44-5), 2-Ethyl-3-hydroxypyran-4-on (4940-11-8), 2-Methyl-3-hydroxypyran-4-on (118-71-8) und Mischungen davon ausgewählt ist.

9. Verwendung einer Kombination von
A) wenigstens einem Phenylglycidat der Formel (1): 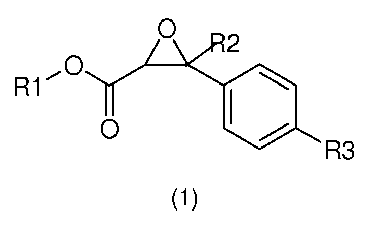 wobei
- R1 eine C₁-C₄-verzweigte oder lineare Alkylgruppe ist,
- R2 ein Wasserstoff oder ein Methyl ist und
- R3 ein Wasserstoff, eine C₁-C₄-verzweigte oder lineare Alkylgruppe oder eine Methoxygruppe ist, und
B) wenigstens einem 1,2-Diketon der Formel (2) oder (3): 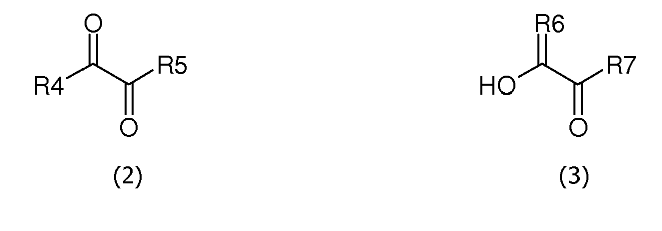 wobei
- R4, R5 und R7 unabhängig voneinander eine C₁-C₅-iineare oder verzweigte Alkyl- oder Alkenylgruppe sein können,
- R6 ein (C₁-C₅)Alkyliden darstellt,
- R4 und R5 auch eine C₄-C₇-gesättigte oder ungesättigte alicyclische oder heterocyclische Ringstruktur bilden können, die durch (C₁-C₄)-Alkylgruppen monosubstituiert oder polysubstituiert sein kann,
- R6 und R7 auch eine C₄-C₇-ungesättigte, alicyclische oder heterocyclische Ringstruktur bilden können, die durch (C₁-C₄)-Alkylgruppen monosubstituiert oder polysubstituiert sein kann,
wobei das Gewichtsverhältnis von Glycidat A zu 1,2-Diketon B zwischen 1:9 und 9:1 liegt;
als Geruchsreduzierendes Mittel.

10. Verwendung nach Anspruch 9, wobei das Phenylglycidat aus Ethyl-3-phenylglycidat (121-39-1), Ethyl-3-methyl-3-phenylglycidat (77-83-8) oder Mischungen davon ausgewählt ist.

11. Verwendung nach Anspruch 9 oder 10, wobei das 1,2-Diketon aus β-Thujaplicin (499-44-5), 3-Methylcyclopent-2-en-2-ol-1-on (765-70-8), 2-Ethyl-3-hydroxypyran-4-on (4940-11-8), 2-Methyl-3-hydroxypyran-4-on (118-71-8), Ethylcycloentenolon (21835-01-8), 3,4-Dimethyl-1,2-cyclopentadion (13494-06-9), 3,5-Dimethyl-1,2-cyclopentadion (13494-07-0), Methyl-2-hydroxy-4-methylcyclopent-2-en-1-on (42348-12-9), 2-Hydroxycyclohex-2-en-1-on (10316-66-2), 1-Methyl-2,3-cyclohexadion (3008-43-3), 1,2-Cyclohexadion (765-87-7) und Mischungen davon ausgewählt ist.

12. Verwendung nach Anspruch 11, wobei das 1,2-Diketon aus β-Thujaplicin (499-44-5), 2-Ethyl-3-hydroxypyran-4-on (4940-11-8), 2-Methyl-3-hydroxypyran-4-on (118-71-8) und Mischungen davon ausgewählt ist.

## Revendications

1. Composition pour réduire les mauvaises odeurs qui consiste en :
A) au moins un phénylglycidate de formule (1): 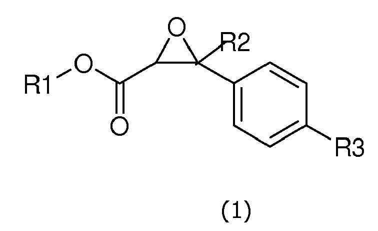 dans laquelle :
- R1 est un groupe alkyle linéaire ou ramifié en C₁-C₄,
- R2 est l'hydrogène ou méthyle, et
- R3 est l'hydrogène, un groupe alkyle linéaire ou ramifié en C₁-C₄, ou un groupe méthoxy, et
B) au moins une 1,2-dicétone de formule (2) ou (3): 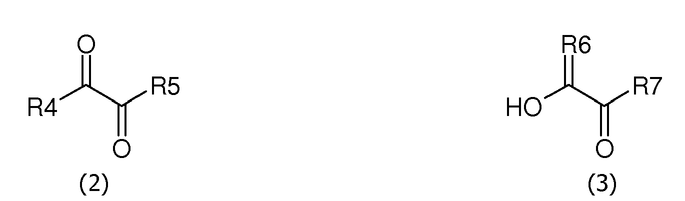 dans laquelle :
- R4, R5 et R7 peuvent être indépendamment un groupe alkyle ou alcényle linéaire ou ramifié en C₁-C₅;
- R6 est un (C₁-C₅)alkylidène;
- R4 et R5 peuvent également former un noyau alicyclique ou hétérocyclique saturé ou insaturé en C₄-C₇, qui peut être mono- ou polysubstitué par des groupes (C₁-C₄)alkyle;
- R6 et R7 peuvent également former un noyau alicyclique ou hétérocyclique insaturé en C₄-C₇, qui peut être mono- ou polysubstitué par des groupes (C₁-C₄)alkyle;
le rapport pondéral du glycidate A à la 1,2 dicétone B étant de 1:9 à 9:1.

2. Composition pour réduire les mauvaises odeurs selon la revendication 1, dans laquelle le phénylglycidate est choisi parmi l'éthyl-3-phénylglycidate (121-39-1), l'éthyl-3-méthyl-3-phénylglycidate (77-83-8) ou leurs mélanges.

3. Composition pour réduire les mauvaises odeurs selon la revendication 1 ou la revendication 2, dans laquelle la 1,2-dicétone est choisie parmi la β thujaplicine (499-44-5), la 3-méthylcyclopent-2-èn-2-ol-1-one (765-70-8), la 2-éthyl-3-hydroxypyran-4-one (4940-11-8), la 2-méthyl-3-hydroxypyran-4-one (118-71-8), l'éthyl cycloentènolone (21835-01-8), la 3,4-diméthyl-1,2-cyclopentadione (13494-06-9), la 3,5-diméthyl-1,2-cyclopentadione (13494-07-0), la méthyl-2-hydroxy-4-méthylcyclopent-2-èn-1-one (42348-12-9), la 2-hydroxycyclohex-2-èn-1-one (10316-66-2), la 1-méthyl-2,3-cyclohexadione (3008-43-3), la 1,2 cyclohexadione (765-87-7) ou leurs mélanges.

4. Composition pour réduire les mauvaises odeurs selon la revendication 3, dans laquelle la 1,2-dicétone est choisie parmi la β thujaplicine (499-44-5), la 2-éthyl-3-hydroxypyran-4-one (4940-11-8), la 2-méthyl-3-hydroxypyran-4-one (118-71-8) ou leurs mélanges.

5. Produit de consommation qui comprend une composition pour réduire les mauvaises odeurs en une quantité de 0,01% à 1,5% en poids de la composition totale dudit produit de consommation, où la composition pour réduire les mauvaises odeurs est une combinaison de :
A) au moins un phénylglycidate de formule (1): 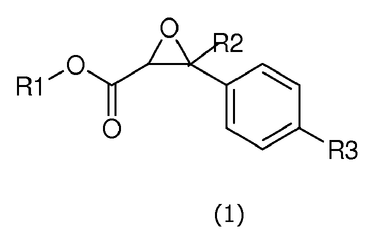 dans laquelle :
- R1 est un groupe alkyle linéaire ou ramifié en C₁-C₄,
- R2 est l'hydrogène ou méthyle, et
- R3 est l'hydrogène, un groupe alkyle linéaire ou ramifié en C₁-C₄ ou un groupe méthoxy, et
B) au moins une 1,2-dicétone de formule (2) ou (3): 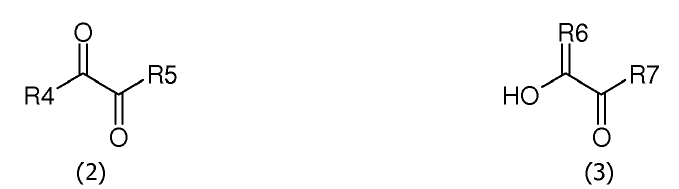 dans laquelle :
- R4, R5 et R7 peuvent être indépendamment un groupe alkyle ou alcényle linéaire ou ramifié en C₁-C₅;
- R6 est un (C₁-C₅)alkylidène;
- R4 et R5 peuvent également former un noyau alicyclique ou hétérocyclique saturé ou insaturé en C₄-C₇, qui peut être mono- ou polysubstitué par des groupes (C₁-C₄)alkyle;
- R6 et R7 peuvent également former un noyau alicyclique ou hétérocyclique insaturé en C₄-C₇, qui peut être mono- ou polysubstitué par des groupes (C₁-C₄)alkyle;
le rapport pondéral du glycidate A à la 1,2 dicétone B étant de 1:9 à 9:1 ;
ledit produit de consommation étant choisi parmi les produits de soin personnels, les détergents à lessive, les assouplissants, les produits de nettoyage ménagers et les produits d'assainissement de l'air.

6. Produit de consommation selon la revendication 5, dans lequel le phénylglycidate est choisi parmi l'éthyl-3-phénylglycidate (121-39-1), l'éthyl-3-méthyl-3-phénylglycidate (77-83-8) ou leurs mélanges.

7. Produit de consommation selon la revendication 5 ou la revendication 6, dans lequel la 1,2-dicétone est choisie parmi la β thujaplicine (499-44-5), la 3-méthylcyclopent-2-èn-2-ol-1-one (765-70-8), la 2-éthyl-3-hydroxypyran-4-one (4940-11-8), la 2-méthyl-3-hydroxypyran-4-one (118-71-8), l'éthyl cycloentènolone (21835-01-8), la 3,4-diméthyl-1,2-cyclopentadione (13494-06-9), la 3,5-diméthyl-1,2-cyclopentadione (13494-07-0), la méthyl-2-hydroxy-4-méthylcyclopent-2-èn-1-one (42348-12-9), la 2-hydroxycyclohex-2-èn-1-one (10316-66-2), la 1-méthyl-2,3-cyclohexadione (3008-43-3), la 1,2 cyclohexadione (765-87-7) ou leurs mélanges.

8. Produit de consommation selon la revendication 7, dans laquelle la 1,2-dicétone est choisie parmi la β thujaplicine (499-44-5), la 2-éthyl-3-hydroxypyran-4-one (4940-11-8), la 2-méthyl-3-hydroxypyran-4-one (118-71-8) ou leurs mélanges.

9. Utilisation d'une combinaison de :
A) au moins un phénylglycidate de formule (1): 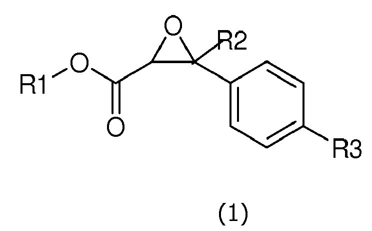 dans laquelle :
- R1 est un groupe alkyle linéaire ou ramifié en C₁-C₄,
- R2 est l'hydrogène ou méthyle, et
- R3 est l'hydrogène, un groupe alkyle linéaire ou ramifié en C₁-C₄ ou un groupe méthoxy, et
B) au moins une 1,2-dicétone de formule (2) ou (3): 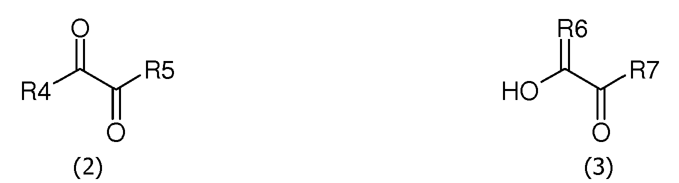 dans laquelle :
- R4, R5 et R7 peuvent être indépendamment un groupe alkyle ou alcényle linéaire ou ramifié en C₁-C₅;
- R6 est un (C₁-C₅)alkylidène;
- R4 et R5 peuvent également former un noyau alicyclique ou hétérocyclique saturé ou insaturé en C₄-C₇, qui peut être mono- ou polysubstitué par des groupes (C₁-C₄)alkyle;
- R6 et R7 peuvent également former un noyau alicyclique ou hétérocyclique insaturé en C₄-C₇, qui peut être mono- ou polysubstitué par des groupes (C₁-C₄)alkyle;
le rapport pondéral du glycidate A à la 1,2 dicétone B étant de 1:9 à 9:1 ;
comme agent réduisant les mauvaises odeurs.

10. Utilisation selon la revendication 9, dans laquelle le phénylglycidate est choisi parmi l'éthyl-3-phénylglycidate (121-39-1), l'éthyl-3-méthyl-3-phénylglycidate (77-83-8) ou leurs mélanges.

11. Utilisation selon la revendication 9 ou la revendication 10, dans laquelle la 1,2-dicétone est choisie parmi la β thujaplicine (499-44-5), la 3-méthylcyclopent-2-èn-2-ol-1-one (765-70-8), la 2-éthyl-3-hydroxypyran-4-one (4940-11-8), la 2-méthyl-3-hydroxypyran-4-one (118-71-8), l'éthyl cycloentènolone (21835-01-8), la 3,4-diméthyl-1,2-cyclopentadione (13494-06-9), la 3,5-diméthyl-1,2-cyclopentadione (13494-07-0), la méthyl-2-hydroxy-4-méthylcyclopent-2-èn-1-one (42348-12-9), la 2-hydroxycyclohex-2-èn-1-one (10316-66-2), la 1-méthyl-2,3-cyclohexadione (3008-43-3), la 1,2 cyclohexadione (765-87-7) ou leurs mélanges.

12. Utilisation selon la revendication 11, dans laquelle la 1,2-dicétone est choisie parmi la β thujaplicine (499-44-5), la 2-éthyl-3-hydroxypyran-4-one (4940-11-8), la 2-méthyl-3-hydroxypyran-4-one (118-71-8) ou leurs mélanges.
